# EUROPEAN PATENT SPECIFICATION

(11) **EP 4 110 209 B1**
(45) Date of publication and mention of the grant of the patent: **21.08.2024**
(21) Application number: 21708618.0
(22) Date of filing: 25.02.2021
(51) Int. Cl.: A61B 17/80, A61B 17/17, A61B 17/70, A61B 90/00

(54) **PLATE HOLDER INSTRUMENT AND KIT COMPRISING THE SAME**
PLATTENHALTERINSTRUMENT UND KIT DAMIT
INSTRUMENT PORTE-PLAQUE ET KIT LE COMPRENANT

(30) Priority: 25.02.2020 US 202062981183 P
(43) Date of publication of application: 04.01.2023
(73) Proprietor: Aesculap AG, 78532 Tuttlingen (DE)
(72) Inventor: CREWS, Robert, Center Valley, Pennsylvania 18034 (US); CORRAO, Ernie, Center Valley, Pennsylvania 18034 (US); LARSEN, Scott, Center Valley, Pennsylvania 18034 (US)
(74) Representative: Winter, Brandl - Partnerschaft mbB
(86) International application number: PCT/EP2021/054732
(87) International publication number: WO 2021/170744

(56) References cited:
- WO-A1-2009/091770
- WO-A1-94/17744
- GB-A- 2 445 146
- US-A- 4 187 840
- US-A1- 2009 018 547
- US-A1- 2010 100 131
- US-A1- 2013 116 733
- US-A1- 2015 238 238

## Description

### TECHNICAL FIELD

The present disclosure relates generally to a plate holder instrument. Moreover, the present disclosure relates to a combination guide and a kit comprising the plate holder instrument and the combination guide.

### BACKGROUND

Spinal fusion is a procedure in which damaged vertebrae are removed, and vertebral bodies adjacent to the removed vertebrae are fused together with graft material. The spine must be immobilized during fusion. To immobilize the spine, one or more fixation rods are anchored to the vertebrae to limit movement.

Cervical-occipito fixation may be accomplished by using fixation rods attached to a bone plate that, in turn, is anchored to the occiput bone on the posterior of the skull. One example of such a bone plate is described in U.S. Patent No. 8,348,981 assigned to Aesculap Implant Systems, LLC.

Occipital bone plates are attached to the skull with bone screws that are inserted through openings in the bone plates. Prior to inserting the screws, the holes for the screws must be drilled and tapped.

Drilling and tapping holes at the rear of the skull is a difficult procedure that requires a significant amount of force to penetrate the dense cortical bone. The process is further complicated by the angle of approach that is required for instruments. In addition, the drill depth must be controlled with precision, which is difficult to do while at the same time applying significant force on the drill at a difficult angle of approach.

Further prior art is known from documents US 2010/100131 A1, US 2013/116733 A1, US 4 187 840 A and WO 2009/091770 A1.

### SUMMARY

The present invention is defined in claim 1 while preferred embodiments are set forth in the dependent claims.

The aforementioned challenges are addressed by instruments in accordance with the present invention.

In accordance with the present invention, a plate holder instrument includes a base having a proximal end and a distal end opposite the proximal end. The base defines a longitudinal axis extending from the proximal end to the distal end in a longitudinal direction. A clamp is displaceable relative to the base in the longitudinal direction and forms a plate receiving recess with the base. The clamp is displaceable between a released position to allow a bone plate to be loaded into and removed from the plate receiving recess, and a clamped position to lock the bone plate in the plate receiving recess. An actuator is displaceable relative to the base in a first direction to move the clamp to the released position and a second direction to move the clamp to the clamped position.

In an embodiment, the plate holder instrument has an actuator with a lever arm pivotally mounted to the proximal end of the base by a hinge.

In another embodiment, the plate holder instrument has an actuator with a link member connected between the lever arm and the clamp.

In another embodiment, the plate holder instrument has an actuator with a link member having a first link end pivotally coupled to a lever arm and a second link end pivotally connected to a clamp.

In another embodiment, the plate holder instrument has a base with a first plate holding surface and a clamp with a second plate holding surface.

In another embodiment, the plate holder instrument has a plate receiving recess that extends between a first plate holding surface and a second plate holding surface.

In another embodiment, the plate holder instrument has a second plate holding surface that is movable away from a first plate holding surface in response to an actuator being moved relative to a base in a first direction.

In another embodiment, the plate holder instrument has a second plate holding surface that is movable toward a first plate holding surface in response to an actuator being moved relative to a base in a second direction.

In another embodiment, the plate holder instrument has a first plate holding surface with a first pair of detents projecting from a base, and a second plate holding surface with a second pair of detents projecting from a clamp.

In another embodiment, the plate holder instrument has a plate receiving recess with a first ramped surface on a first side of a longitudinal axis and a second ramped surface on a second side of the longitudinal axis, the second ramped surface being non parallel to the first ramped surface.

In another embodiment, the plate holder instrument has a base with at least one stop surface and a clamp that has at least one stop member configured to abut the at least one stop surface when the clamp is moved to a clamped position.

In another embodiment, the plate holder instrument has a base and clamp that collectively define an aperture adjacent a plate receiving recess, the aperture configured to extend behind a plate secured in the plate receiving recess.

In accordance with the present invention, the plate holder instrument has a fastener to detachably couple a guide accessory to the plate holder instrument.

In another embodiment, the plate holder instrument has a fastener in the form of a clip.

In another embodiment, the plate holder instrument has a clamp that is displaceable relative to a base to a cleaning position in which the clamp is separated from the base to facilitate sterilization.

In another embodiment, the plate holder instrument has an actuator is movable in the first direction to move the clamp from the released position to the cleaning position.

Separately or additionally to the plate holder instrument as defined above, in an example of the disclosure, a combination guide preferably for drilling and tapping guides configured to be coupled to a plate holder instrument is provided. The combination guide comprises a first end (portion), a second end (portion) and an elongated body extending between the first end (portion) and the second end (portion). The first end (portion) is configured to serve as a drilling guide, and the second end (portion) is configured to serve as a tapping guide. In this way, tapping and drilling guides can be realized through a single combination guide with a simple and compact structure.

In the following, only the expression "end" is used, wherein also "end portion" is alternatively intended.

In another example, the first end of a combination guide has a cylindrical guide ring adapted to receive a drill bit and a second end of the combination guide has a cylindrical guide ring adapted to receive a tapping tool.

In another example, the first end of a combination guide is connected to an elongated body of the combination guide through a dog leg section and a second end of the combination guide is connected to the elongated body of the combination guide through a second dog leg section.

In accordance with the present invention, a kit for attaching a bone plate to a posterior area of a patient's skull is provided. The kit comprises a plate holder instrument for holding the bone plate according to any one of claims 1 to 14. In an example of the present disclosure, the kit also comprises a combination guide preferably for drilling and tapping guides. In addition, the kit preferably comprises a bone plate.

In another example of the disclosure, a kit for attaching a bone plate to a posterior area of a patient's skull includes a plate holder instrument. The plate holder instrument includes a base having a proximal end and a distal end opposite the proximal end. The base defines a longitudinal axis extending from the proximal end to the distal end in a longitudinal direction. A clamp is displaceable relative to the base in the longitudinal direction and forms a plate receiving recess with the base. The clamp is displaceable between a released position to allow a bone plate to be loaded into and removed from the plate receiving recess, and a clamped position to lock a plate in the plate receiving recess. An actuator is displaceable relative to the base in a first direction to move the clamp to the released position and a second direction to move the clamp to the clamped position. At least one guide accessory is configured to be detachably coupled to the plate holder instrument.

In another example of the disclosure, a kit has at least one guide accessory in the form of at least one drill guide.

In another example of the disclosure, a kit has at least one guide accessory in the form of at least one tap guide.

In another example of the disclosure, a kit has at least one bone plate.

### BRIEF DESCRIPTION OF THE DRAWING FIGURES

The foregoing summary and the following detailed description will be better understood in conjunction with non-limiting examples shown in the drawing figures, of which:
FIG. 1 is a schematic perspective view of a cervical-occipito fixation system featuring a bone plate attached to the posterior of a patient's skull;
FIG. 2 is a front perspective view of a plate holder instrument according the present invention;
FIG. 3 is a rear perspective view of the plate holder instrument of FIG. 2;
FIG. 4 is another front perspective view of the plate holder instrument of FIG. 2 with a bone plate loaded into the plate holder instrument;
FIG. 5 is another front perspective view of the plate holder instrument of FIG. 2 with the bone plate clamped in the plate holder instrument;
FIG. 6 is an enlarged truncated perspective view of the plate holder instrument of FIG. 2, showing details at one end;
FIG. 7 is another enlarged truncated perspective view of the plate holder instrument of FIG. 2, with a bone plate secured in one end;
FIG. 8 is a front perspective view of a combination guide according a first example of the present disclosure;
FIG. 9 is a front perspective view of a combination guide according a second example of the present disclosure;
FIG. 10 is a front perspective view of the combination guide according to the first example attached to the plate holder instrument of FIG. 2 and the bone plate according to the present disclosure;
FIG. 11 is a rear perspective view of the combination guide according to the first example attached to the plate holder instrument and the bone plate of FIG. 10;
FIG. 12 is a schematic side view of the combination guide according to the first example attached to the plate holder instrument and the bone plate of FIG. 10, shown during drilling of a screw hole;
FIG. 13 is an enlarged perspective view showing details of the combination guide according to the first example, the plate holder instrument, and the bone plate during drilling of a screw hole;
FIG. 14 is an enlarged perspective view showing details of the combination guide according to the first example, the plate holder instrument, and the bone plate during tapping of a screw hole;
FIG. 15 is an enlarged perspective view showing details of the plate holder instrument and the bone plate during insertion of a screw through the bone plate and into a tapped screw hole;
FIG. 16 is a perspective view of another plate holder instrument and the bone plate attached to a combination guide according to a third example of the present disclosure;
FIG. 17 is a schematic perspective view of the plate holder instrument, the plate and the combination guide of FIG. 16 with a tapping instrument;
FIG. 18 is a schematic perspective view of a plate holder instrument according to the present invention being detached from the bone plate after the bone plate is anchored to a patient's occipital bone;
FIG. 19 is another front perspective view of the plate holder instrument of FIG. 2, in which the plate is removed and the actuator is moved to a cleaning position to permit components to be sterilized; and
FIG. 20 is a perspective view of another plate holder instrument according to the present disclosure.

### DETAILED DESCRIPTION

The following section describes different instruments used for fixation of the cervical spine according to the present disclosure.

In the present disclosure, "distal" basically means "in a direction away from a user/ surgeon towards a patient" and "proximal" basically means "in a direction towards the user/ surgeon away from the patient".

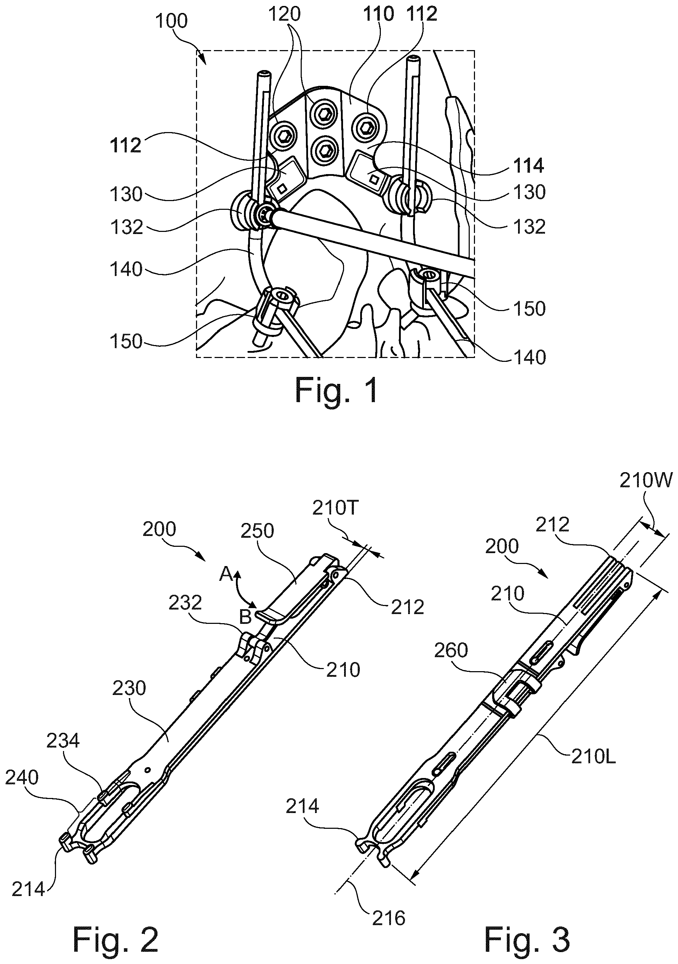

FIG. 1 shows a cervical-occipito fixation system 100 featuring a bone plate 110 attached to the occipital bone of a patient's skull. The bone plate 110 is anchored to the occipital bone with four bone screws 120. The bone screws 120 are driven through holes 112 in a main body portion 114 of the bone plate. A pair of legs 130 extend outwardly from main body portion 114. Each leg 130 has a socket 132 configured to support a cervical fixation rod 140. The cervical fixation rods 140 are connected to bone anchor assemblies 150 implanted in the spine.

FIGS. 2 and 3 show a plate holder instrument 200. The plate holder instrument 200 is configured to hold a bone plate in a desired position where it is to be attached to an occipital bone. The plate holder instrument 200 also works with other accessories, as will be described, to drill and tap screw holes in the occipital bone. Moreover, the plate holder instrument 200 can be used to hold the bone plate in position as bone screws are driven through the tapped screw holes to anchor the bone plate to the occipital bone.

The plate holder instruments according to the present disclosure have components that move relative to one another to engage and disengage a bone plate. The components can have various structures. Relatively long and thin components with small thicknesses allow the plate holder instrument to have a long and thin profile so that the instrument can access the occipital bone while taking up minimal space. In the present example, the plate holder instrument 200 has a plate-shaped base 210 with an axial length 210L, width 210W, and thickness 210T. The thickness 210T is a small fraction of the width 210W, and the width 210W is a small fraction of the axial length 210L. The base 210 has a proximal end 212 and a distal end 214 opposite the proximal end 212. The base 210 also defines a longitudinal axis 216 extending from the proximal end 212 to the distal end 214 in a longitudinal direction.

The plate holder instrument 200 includes a plate-shaped clamp 230. The clamp 230 has a proximal end 232 and a distal end 234 opposite the proximal end 232. In addition, the clamp 230 is connected to the base 210 in an axially displaceable arrangement. In this arrangement, the clamp 230 is displaceable relative to the base 210 in the longitudinal direction. The clamp 230 forms a plate receiving recess 240 with the base 210. The plate receiving recess 240 is sized to receive and support a bone plate. The clamp 230 is displaceable relative to the base 210 between a released position and a clamped position. In the released position, the clamp 230 allows a bone plate, like a bone plate 110, to be loaded into and removed from the plate receiving recess 240. In the clamped position, the clamp 230 securely engages a bone plate 110 in the plate receiving recess 240, so that the bone plate 110 is locked in the plate receiving recess 240.

The Plate holder instrument 200 further includes an actuator 250. The actuator 250 is displaceable relative to the base 210 in a first direction A to move the clamp 230 to the released position. The actuator 250 is also displaceable relative to the base 210 in a second direction B to move the clamp 230 to the clamped position.

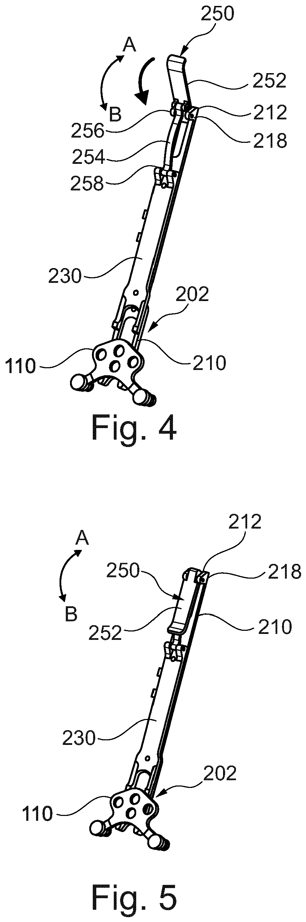

FIGS. 4 and 5 show the plate holder instrument 200 with the bone plate 110 loaded into the plate receiving recess 240. When the actuator 250 is moved in the first direction A as shown in FIG. 4, the clamp 230 is moved to the released position. In this position, the plate receiving recess 240 is expanded, providing clearance to allow the loading of the bone plate 110 into the plate receiving recess 240 between the base 210 and the clamp 230. When the actuator 250 is moved in the second direction B as shown in FIG. 5, the clamp 230 is moved to the clamped position. In this position, the plate receiving recess 240 is contracted or reduced in size to enclose the bone plate 110 on two sides. In this arrangement, the base 210 and the clamp 230 collectively form a vise 202 that can be opened and closed via the actuator 250 to unclamp or clamp a bone plate 110.

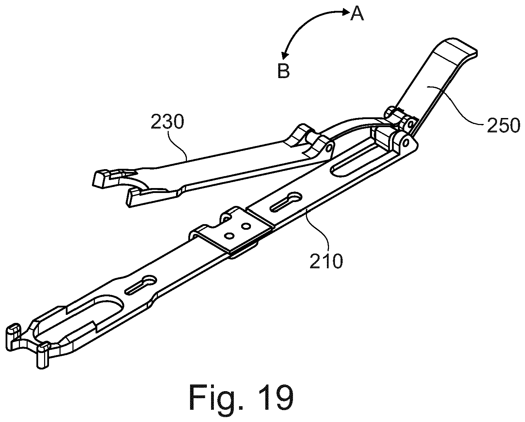

The actuator 250 can be moved in the first direction A beyond the released position to a cleaning position, as shown in FIG. 19. In this position, the clamp 230 is detached from the base 210, which allows the components to be sterilized.

Referring back to FIGS. 4 and 5, the actuator 250 includes a lever arm 252 pivotally mounted to the proximal end 212 of the base 210 by a hinge 218. The actuator 250 further includes a link member 254 connected between the lever arm 252 and the clamp 230. The link member 254 has a first link end 256 pivotally coupled to the lever arm 252 and a second link end 258 pivotally connected to the clamp 230.

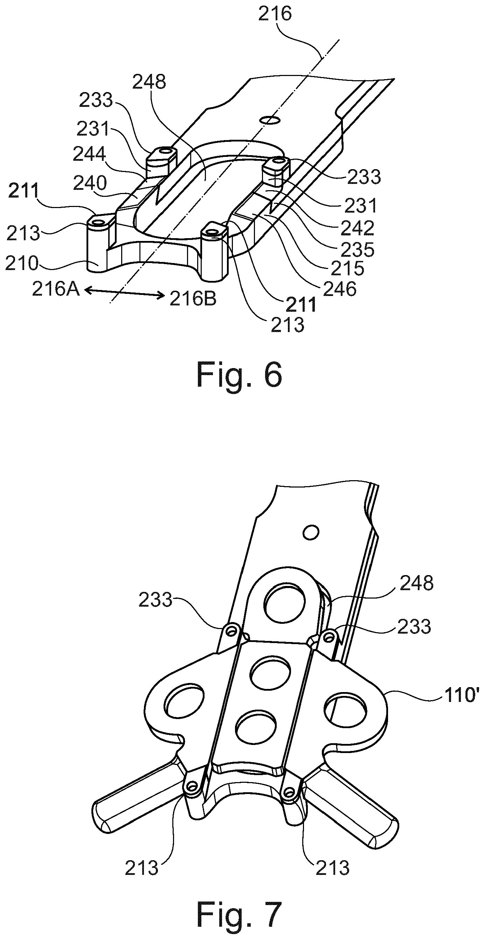

Referring to FIG. 6, the base 210 includes a first plate holding surface 211 and the clamp 230 includes a second plate holding surface 231. The plate receiving recess 240 extends between the first plate holding surface 211 and the second plate holding surface 231. The second plate holding surface 231 is movable away from the first plate holding surface 211 in response to the actuator 250 being moved relative to the base 210 in the first direction A. The second plate holding surface 231 is movable toward the first plate holding surface 211 in response to the actuator 250 being moved relative to the base 210 in the second direction B.

The occipital bone typically has a curvature. The bone plates with a flat profile are difficult to secure to a curved bone surface. Therefore, it is often desirable for the bone plate to be bent or otherwise shaped so as to have a curved contour or a face that conforms to the natural curvature of the occipital bone. For example, the plate can be bent so that the side of the plate to be secured against the occipital bone has a concave shape.

Plate holder instruments and plate receiving recesses according to the present disclosure can have one or more surfaces that conform to the curvature of curved bone plates. Referring to FIG. 7, the plate receiving recess 240 has a contoured support surface 242 that conforms to the curved rear face of a bone plate 110'. In particular, the plate receiving recess 240 includes a first ramped surface 244 on a first side 216A of longitudinal axis 216. The plate receiving recess 240 also includes a second ramped surface 246 on a second side 216B of the longitudinal axis 216. The second ramped surface 246 is non parallel to the first ramped surface 244. Moreover, the first ramped surface 244 and the second ramped surface 246 are inclined toward one another as they extend toward the longitudinal axis 216. In this arrangement, the first and second ramped surfaces 244, 246 collectively form a V-shaped or U-shaped support surface 246 that conforms to the curvature of the bone plate 110'.

The first plate holding surface 211 includes a first pair of detents 213 projecting from the base 210. Similarly, the second plate holding surface 231 includes a second pair of detents 233 projecting from the clamp 230. The first pair of detents 213 and the second pair of detents 233 only contact the bone plate 110' at four locations along the perimeter of the bone plate.

Plate holder instruments according to the present disclosure can include stop mechanisms for limiting displacement of the clamp relative to the bone plate as the bone plate is moved to the clamped position. The stop mechanisms can be used to prevent a bone plate from being clamped too tightly, potentially deforming and damaging the bone plate. In the present example, the base 210 includes a pair of stop surfaces 215 that face toward proximal end 212 of the base 210. In addition, the clamp 230 includes a pair of stop members 235 that define distal end 234 of the clamp 230. The stop members 235 are configured to abut the stop surfaces 215 when the clamp 230 is moved to the clamped position, preventing the second plate holding surface 231 from moving any closer to the first plate holding surface 211.

The base 210 and the clamp 230 collectively define an aperture 248 adjacent to the plate receiving recess 240. The aperture 248 is configured to extend behind the holes of a bone plate secured in the plate receiving recess 240, as shown for example in FIG. 7, so that a clear unobstructed path extends through the holes in the bone plate to occipital bone when the plate holder instrument and bone plate are held against the occipital bone.

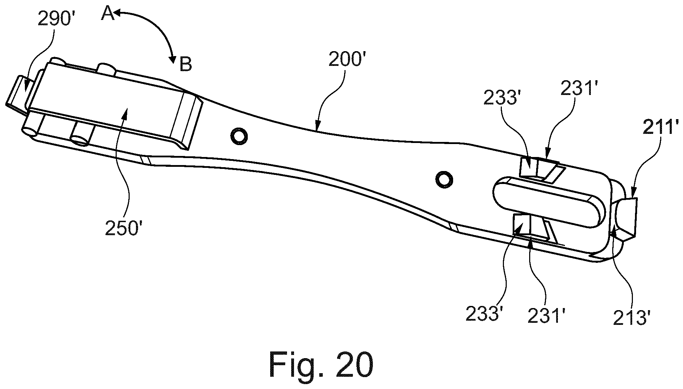

Plate holder instruments according to the present disclosure can have various plate holding structures and configurations. For example, a plate holding surface can be comprised of a single detent, rather than a pair of detents. Referring to FIG. 20, a plate holder 200' is shown with a first plate holding surface 211' comprised of a single detent 213', and a second plate holding surface 231' with a pair of detents 233'. In this arrangement, the single detent 213' and the pair of detents 233' contact a bone plate at three locations along the perimeter of the bone plate. The plate holder 200' also includes a guard 290' that limits how far an actuator 250' can be pivoted in the first direction A.

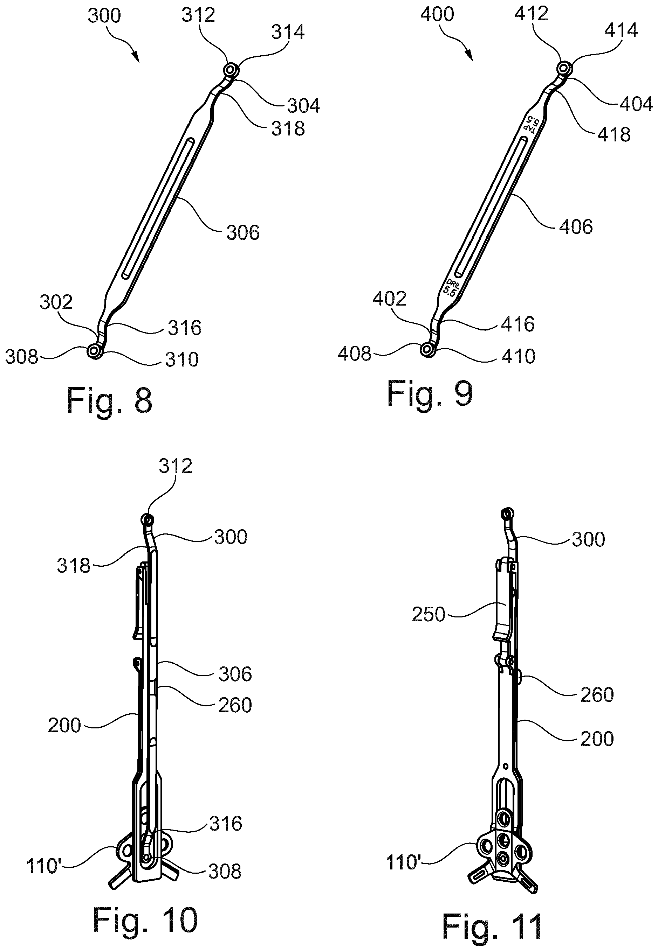

Plate holder instruments according to the present disclosure are configured to work with different accessories, including guides that aid in the drilling and tapping of screw holes in the occipital bone, and guides for driving screws through a bone plate. In the present example, the plate holder instrument 200 includes a fastener in the form of a clip 260, the rear facing portion of which is shown in FIGS. 3 and 10. The clip 260 is configured to detachably couple a guide accessory to the plate holder instrument.

Plate holder instruments according to the present disclosure can be marketed as stand-alone instruments. Alternatively, plate holder instruments according to the present disclosure can be marketed with one or more accessories. Moreover, plate holder instruments according to the present disclosure can be marketed as part of a surgical set that includes various surgical instruments, bone plates, screws, tools, taps, depth gauges, screw drivers and other accessories.

For example, kits according to the present disclosure can include accessories for drilling pilot holes in the occipital bone. Such accessories can include surgical drills, drill bits, and drill guides. Kits according to the present disclosure can also include accessories for tapping screw holes in the occipital bone. Tapping screw holes can involve driving a tapping instrument into a pilot hole to form threading in the screw hole. Thus, kits according to the present disclosure can include taps and tapping guides.

Kits according to the present disclosure can also include one or more bone plates. For example, a kit can include a set of bone plates, with the bone plates differing in terms of their dimensions, curvatures, hole diameters, hole arrangements and/or other design parameters.

Guide accessories according to the present disclosure can include combination guides featuring a first end for guiding the drilling of a pilot hole, and a second end for guiding the tapping of screw hole. FIG. 8 shows one example of a combination guide 300 used for tapping a screw hole to receive a 4.5 mm screw in a mini-access procedure. The combination guide 300 has a first end 302, a second end 304 and an elongated body 306 extending between the first end 302 and second end 304. The first end 302 has a cylindrical guide ring 308 adapted to receive a drill bit, so as to serve as a drill guide 310. The second end 304 has a cylindrical guide ring 312 adapted to receive a tapping tool, so as to serve as a tapping guide 314. The first end 302 is connected to the elongated body 306 by a first dog leg section 316, and the second end 304 is connected to the elongated body 306 by a second dog leg section 318.

FIG. 9 shows an example of another combination guide 400 used for tapping a screw to receive a 5.5 mm screw hole in a mini-access procedure. The combination guide 400 has a first end 402, a second end 404 and an elongated body 406 extending between the first end 402 and second end 404. The first end 402 has a cylindrical guide ring 408 adapted to receive a drill bit, so as to serve as a drill guide 410. The second end 404 has a cylindrical guide ring 412 adapted to receive a tapping tool, so as to serve as a tapping guide 414. The first end 402 is connected to the elongated body 406 by a first dog leg section 416, and the second end 404 is connected to the elongated body 406 by a second dog leg section 418.

The elongated bodies 306, 406 of the combination guides 300, 400 are configured to be inserted into the clip 260 of the plate holder instrument 200 and releasably retained by the clip 260. Referring to FIGS. 10 and 11, the plate holder instrument 200 is shown with the combination guide 300 inserted in the clip 260 and aligned with a bone plate 110' loaded in the plate holder instrument 200. It will be appreciated that the combination guide 400 can be inserted in the clip 260 and used with the plate holder instrument 200 and the bone plate 110' in the same manner. Therefore, only the structural and functional aspects of the combination guide 300 will be described, with the understanding that the structural and functional aspects of the combination guide 400 are identical in all respects, with the only difference being the size of the guide rings.

The first and second dog leg sections 316, 318 create offsets that allow the guide rings 308, 312 to be positioned flush against the bone plate 110', while the elongated body 306 is positioned outwardly from the bone plate 110'. These offsets are necessary to allow the guide rings 308, 312 to be positioned as close to bone plate 110' as possible, while allowing the elongated body 306 to be coupled over top of the plate holder instrument 200 in a straddling arrangement.

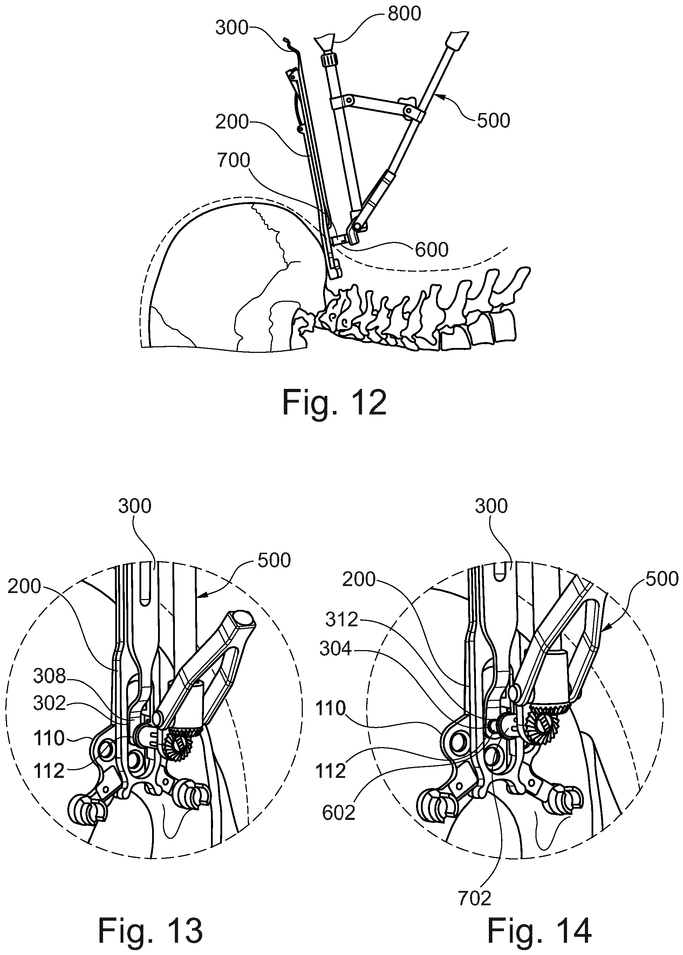

Plate holder instruments according to the present disclosure can also be used with angled instruments for guiding surgical drills and tapping instruments. FIGS. 12 and 13 show the plate holder instrument 200 holding the bone plate 110 against the occipital bone during the drilling of a pilot hole. An angled gear instrument 500 with a drill bit 600 and a depth stop 700 drill the pilot hole, with torque provided by a drill driver 800. In this instance, the combination guide 300 is coupled to the plate holder instrument 200 with the guide ring 308 on the first end 302 aligned with a hole 112 in the bone plate 110.

FIG. 14 shows the plate holder instrument 200 holding the bone plate 110 against the occipital bone during tapping of the screw hole. The angled gear instrument 500 has a tap 602 and depth stop 702. In this instance, the combination guide 300 is coupled to the plate holder 200 with the guide ring 312 on the second end 304 aligned with a hole 112 in the bone plate 110.

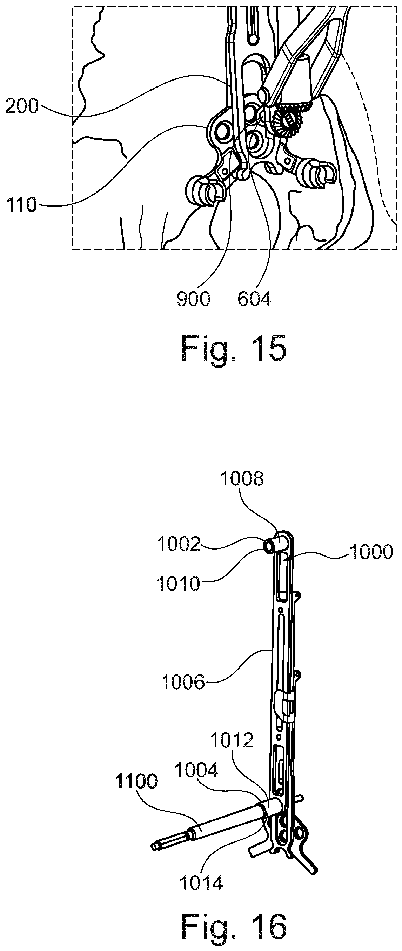

FIG. 15 shows the plate holder instrument 200 holding the bone plate 110 against the occipital bone as the bone plate 110 is anchored to the occipital bone. In this instance, the tapped screw hole is prepared, and the combination guide 300 is therefore removed from the plate holder 200. A first plate screw 900 is placed on a driver bit 604 attached to the angled gear instrument 500. Torque applied to the angled gear instrument 500 drives the first plate screw 900 through a first hole in the bone plate 110 and into a tapped hole to anchor the bone plate 110 to the occipital bone. Once the first plate screw 900 is driven into the screw hole, the angled gear instrument 500 can be detached, loaded with a second plate screw, and maneuvered to a second hole in the bone plate 110 to drive the second plate screw through the second hole and into the occipital bone. This process can be repeated for any other screw holes that are tapped behind bone plate 110.

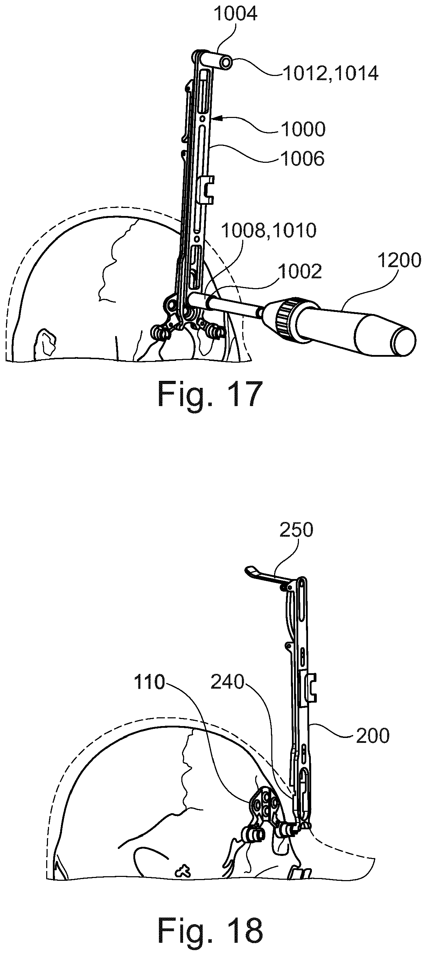

FIGS. 16 and 17 show an alternate combination guide 1000 that can be used in an open procedure. The combination guide 1000 has a first end 1002, a second end 1004 and an elongated body 1006 extending between the first end 1002 and second end 1004. The first end 1002 has a cylindrical guide ring 1008 adapted to receive either a drill bit or a tap bit. The second end 1004 also has a cylindrical guide ring 1012 adapted to receive either a drill bit or tap bit. FIG. 16 shows a tap bit 1100 in the guide ring 1012, and FIG. 17 shows a tap bit 1200 in the guide ring 1008. Unlike the combination guides 300, 400, the combination guide 1000 does not have dog leg sections between the elongated body 1006 and guide rings.

FIG. 18 shows bone plate 110 anchored to the occipital bone. The actuator 250 of the plate holder instrument 200 is moved to the release position to disengage thte bone plate 110 from the plate receiving recess 240.

The instruments described herein can be manufactured using various materials, including but not limited to various alloys of stainless steel. Alloy grade can be selected based on desired strength, hardness, corrosion resistance, galling properties and other performance criteria.

## Claims

1. A plate holder instrument (200) comprising:
a base (210) having a proximal end (212) and a distal end (214) opposite the proximal end (212), the base (210) defining a longitudinal axis (216) extending from the proximal end (212) to the distal end (214) in a longitudinal direction;
a clamp (230) displaceable relative to the base (210) in the longitudinal direction and forming a plate receiving recess (240) with the base (210), the clamp (230) being displaceable between a released position to allow a bone plate (110) to be loaded into and removed from the plate receiving recess (240), and a clamped position to lock the bone plate (110) in the plate receiving recess (240); and
an actuator (250) displaceable relative to the base (210) in a first direction to move the clamp (230) to the released position and a second direction to move the clamp (230) to the clamped position,
**characterised in that** the plate holder instrument further comprises a fastener to detachably couple a guide accessory to the plate holder instrument (200).

2. The plate holder instrument (200) of claim 1, wherein the actuator (250) comprises a lever arm (252) pivotally mounted to the proximal end (212) of the base (210) by a hinge (218).

3. The plate holder instrument (200) of claim 2, wherein the actuator (250) further comprises a link member (254) connected between the lever arm (252) and the clamp (230).

4. The plate holder instrument (200) of claim 3, wherein the link member (254) has a first link end (256) pivotally coupled to the lever arm (252) and a second link end (258) pivotally connected to the clamp (230).

5. The plate holder instrument (200) according to any of the preceding claims 1 to 4, wherein the base (210) comprises a first plate holding surface (211) and the clamp (230) comprises a second plate holding surface (231).

6. The plate holder instrument (200) of claim 5, wherein the plate receiving recess (240) extends between the first plate holding surface (211) and the second plate holding surface (231).

7. The plate holder instrument (200) of claim 5 or 6, wherein the second plate holding surface (231) is movable away from the first plate holding surface (211) in response to the actuator (250) being moved relative to the base (210) in the first direction.

8. The plate holder instrument (200) according to any one of claims 5 to 7, wherein the second plate holding surface (231) is movable toward the first plate holding surface (211) in response to the actuator (250) being moved relative to the base (210) in the second direction.

9. The plate holder instrument (200) according to any one of claims 5 to 8, wherein the first plate holding surface (211) comprises a first pair of detents (213) projecting from the base (210), and the second plate holding surface (231) comprises a second pair of detents (233) projecting from the clamp (230).

10. The plate holder instrument (200) according to any one claims 1 to 9, wherein the plate receiving recess (240) comprises a first ramped surface (244) on a first side (216A) of the longitudinal axis (216) and a second ramped surface (246) on a second side (216B) of the longitudinal axis (216), the second ramped surface (246) being non parallel to the first ramped surface (244).

11. The plate holder instrument (200) according to any one of the preceding claims 1 to 10, wherein the base (210) comprises at least one stop surface (215) and wherein the clamp (230) comprises at least one stop member (235) configured to abut the stop surface (215) when the clamp (230) is moved to the clamped position.

12. The plate holder instrument (200) of claim 1, wherein the fastener comprises a clip (260).

13. The plate holder instrument (200) according to any one of the preceding claims 1 to 12, wherein the clamp (230) is further displaceable relative to the base (210) to a cleaning position in which the clamp (230) is separated from the base (210) to facilitate sterilization.

14. The plate holder instrument (200) of claim 13, wherein the actuator (250) is movable in the first direction to move the clamp (230) from the released position to the cleaning position.

15. A kit for attaching a bone plate to a posterior area of a patient's skull, the kit comprising:
a plate holder instrument (200) according to any one of claims 1 to 14.

## Patentansprüche

1. Plattenhalterinstrument (200) mit:
einer Basis (210) mit einem proximalen Ende (212) und einem distalen Ende (214) gegenüber dem proximalen Ende (212), wobei die Basis (210) eine Längsachse (216) definiert, die sich von dem proximalen Ende (212) zu dem distalen Ende (214) in einer Längsrichtung erstreckt;
einer Klammer (230), die relativ zur Basis (210) in Längsrichtung verschiebbar ist und mit der Basis (210) eine Plattenaufnahmevertiefung (240) bildet, wobei die Klammer (230) verschiebbar ist zwischen einer gelösten Position, in der eine Knochenplatte (110) in die Plattenaufnahmevertiefung (240) geladen und daraus entfernt werden kann, und einer geklemmten Position, in der die Knochenplatte (110) in der Plattenaufnahmevertiefung (240) verriegelt ist; und
einem Aktuator (250), der relativ zur Basis (210) in einer ersten Richtung verschiebbar ist, um die Klammer (230) in die gelöste Position zu bewegen, und in einer zweiten Richtung, um die Klammer (230) in die eingespannte Position zu bewegen,
**dadurch gekennzeichnet, dass**
das Plattenhalterinstrument ferner einen Verschluss umfasst, um ein Führungszubehörteil abnehmbar mit dem Plattenhalterinstrument (200) zu verbinden.

2. Plattenhalterinstrument (200) nach Anspruch 1, wobei der Aktuator (250) einen Hebelarm (252) umfasst, der über ein Scharnier (218) schwenkbar am proximalen Ende (212) der Basis (210) angebracht ist.

3. Plattenhalterinstrument (200) nach Anspruch 2, wobei der Aktuator (250) ferner ein Verbindungsteil (254) umfasst, das zwischen dem Hebelarm (252) und der Klammer (230) angeschlossen ist.

4. Plattenhalterinstrument (200) nach Anspruch 3, wobei das Verbindungsteil (254) ein erstes Verbindungsende (256) aufweist, das schwenkbar mit dem Hebelarm (252) verbunden ist, und ein zweites Verbindungsende (258), das schwenkbar mit der Klammer (230) verbunden ist.

5. Plattenhalterinstrument (200) nach einem der vorhergehenden Ansprüche 1 bis 4, wobei die Basis (210) eine erste Plattenhaltefläche (211) und die Klammer (230) eine zweite Plattenhaltefläche (231) umfasst.

6. Plattenhalterinstrument (200) nach Anspruch 5, wobei sich die Plattenaufnahmevertiefung (240) zwischen der ersten Plattenhaltefläche (211) und der zweiten Plattenhaltefläche (231) erstreckt.

7. Plattenhalterinstrument (200) nach Anspruch 5 oder 6, wobei die zweite Plattenhaltefläche (231) von der ersten Plattenhaltefläche (211) wegbewegt werden kann, wenn der Aktuator (250) relativ zur Basis (210) in die erste Richtung bewegt wird.

8. Plattenhalterinstrument (200) nach einem der Ansprüche 5 bis 7, wobei die zweite Plattenhaltefläche (231) in Richtung der ersten Plattenhaltefläche (211) bewegt werden kann, wenn der Aktuator (250) relativ zur Basis (210) in die zweite Richtung bewegt wird.

9. Plattenhalterinstrument (200) nach einem der Ansprüche 5 bis 8, wobei die erste Plattenhaltefläche (211) ein erstes Paar von Arretierungen (213) umfasst, die von der Basis (210) vorstehen, und die zweite Plattenhaltefläche (231) ein zweites Paar von Arretierungen (233) umfasst, die von der Klammer (230) vorstehen.

10. Plattenhalterinstrument (200) nach einem der Ansprüche 1 bis 9, wobei die Plattenaufnahmevertiefung (240) eine erste geneigte Fläche (244) auf einer ersten Seite (216A) der Längsachse (216) und eine zweite geneigte Fläche (246) auf einer zweiten Seite (216B) der Längsachse (216) umfasst, wobei die zweite geneigte Fläche (246) nicht parallel zu der ersten geneigten Fläche (244) ist.

11. Plattenhalterinstrument (200) nach einem der vorhergehenden Ansprüche 1 bis 10, wobei die Basis (210) mindestens eine Anschlagfläche (215) umfasst und wobei die Klammer (230) mindestens ein Anschlagelement (235) umfasst, das so konfiguriert ist, dass es an der Anschlagfläche (215) anliegt, wenn die Klammer (230) in die eingespannte Position bewegt wird.

12. Plattenhalterinstrument (200) nach Anspruch 1, wobei das Befestigungsmittel einen Clip (260) umfasst.

13. Plattenhalterinstrument (200) nach einem der vorhergehenden Ansprüche 1 bis 12, wobei die Klammer (230) ferner relativ zur Basis (210) in eine Reinigungsposition verschiebbar ist, in der die Klammer (230) von der Basis (210) getrennt ist, um die Sterilisation zu erleichtern.

14. Plattenhalterinstrument (200) nach Anspruch 13, wobei der Aktuator (250) in der ersten Richtung beweglich ist, um die Klammer (230) aus der gelösten Position in die Reinigungsposition zu bewegen.

15. Kit zum Anbringen einer Knochenplatte an einem hinteren Bereich des Schädels eines Patienten, wobei der Bausatz Folgendes umfasst:
ein Plattenhalterinstrument (200) nach einem der Ansprüche 1 bis 14.

## Revendications

1. Instrument porte-plaque (200) comprenant :
une base (210) présentant une extrémité proximale (212) et une extrémité distale (214) opposée à l'extrémité proximale (212), la base (210) définissant un axe longitudinal (216) s'étendant de l'extrémité proximale (212) à l'extrémité distale (214) dans une direction longitudinale ;
une pince (230) déplaçable par rapport à la base (210) dans la direction longitudinale et formant un évidement de réception de plaque (240) avec la base (210), la pince (230) étant déplaçable entre une position libérée pour permettre à une plaque vissée (110) d'être chargée dans et retirée de l'évidement de réception de plaque (240), et une position serrée pour verrouiller la plaque vissée (110) dans l'évidement de réception de plaque (240) ; et
un actionneur (250) déplaçable par rapport à la base (210) dans une première direction pour déplacer la pince (230) vers la position libérée et dans une seconde direction pour déplacer la pince (230) vers la position serrée,
**caractérisé en ce que** l'instrument porte-plaque comprend en outre une attache pour coupler de manière détachable un accessoire de guidage à l'instrument porte-plaque (200).

2. Instrument porte-plaque (200) selon la revendication 1, dans lequel l'actionneur (250) comprend un bras de levier (252) monté de manière pivotante sur l'extrémité proximale (212) de la base (210) par une charnière (218).

3. Instrument porte-plaque (200) selon la revendication 2, dans lequel l'actionneur (250) comprend en outre un élément de liaison (254) monté entre le bras de levier (252) et la pince (230).

4. Instrument porte-plaque (200) selon la revendication 3, dans lequel l'élément de liaison (254) présente une première extrémité de liaison (256) couplée de manière pivotante au bras de levier (252) et une seconde extrémité de liaison (258) reliée de manière pivotante à la pince (230).

5. Instrument porte-plaque (200) selon l'une quelconque des revendications 1 à 4 précédentes, dans lequel la base (210) comprend une première surface de maintien de plaque (211) et la pince (230) comprend une seconde surface de maintien de plaque (231).

6. Instrument porte-plaque (200) selon la revendication 5, dans lequel l'évidement de réception de plaque (240) s'étend entre la première surface de maintien de plaque (211) et la seconde surface de maintien de plaque (231).

7. Instrument porte-plaque (200) selon la revendication 5 ou 6, dans lequel la seconde surface de maintien de plaque (231) peut s'éloigner de la première surface de maintien de plaque (211) en réponse au déplacement de l'actionneur (250) par rapport à la base (210) dans la première direction.

8. Instrument porte-plaque (200) selon l'une quelconque des revendications 5 à 7, dans lequel la seconde surface de maintien de plaque (231) est mobile vers la première surface de maintien de plaque (211) en réponse au déplacement de l'actionneur (250) par rapport à la base (210) dans la seconde direction.

9. Instrument porte-plaque (200) selon l'une quelconque des revendications 5 à 8, dans lequel la première surface de maintien de plaque (211) comprend une première paire d'ergots (213) faisant saillie depuis la base (210), et la seconde surface de maintien de plaque (231) comprend une seconde paire d'ergots (233) faisant saillie depuis la pince (230).

10. Instrument porte-plaque (200) selon l'une quelconque des revendications 1 à 9, dans lequel l'évidement de réception de plaque (240) comprend une première surface inclinée (244) sur un premier côté (216A) de l'axe longitudinal (216) et une seconde surface inclinée (246) sur un second côté (216B) de l'axe longitudinal (216), la seconde surface inclinée (246) n'étant pas parallèle à la première surface inclinée (244).

11. Instrument porte-plaque (200) selon l'une quelconque des revendications 1 à 10 précédentes, dans lequel la base (210) comprend au moins une surface d'arrêt (215) et dans lequel la pince (230) comprend au moins un élément d'arrêt (235) configuré pour venir en butée contre la surface d'arrêt (215) lorsque la pince (230) est déplacée vers la position serrée.

12. Instrument porte-plaque (200) selon la revendication 1, dans lequel l'attache comprend un clip (260).

13. Instrument porte-plaque (200) selon l'une quelconque des revendications 1 à 12 précédentes, dans lequel la pince (230) est en outre déplaçable par rapport à la base (210) vers une position de nettoyage dans laquelle la pince (230) est séparée de la base (210) pour faciliter la stérilisation.

14. Instrument porte-plaque (200) selon la revendication 13, dans lequel l'actionneur (250) est mobile dans la première direction pour déplacer la pince (230) de la position libérée à la position de nettoyage.

15. Kit pour fixer une plaque vissée à une zone postérieure du crâne d'un patient, le kit comprenant :
un instrument porte-plaque (200) selon l'une quelconque des revendications 1 à 14.
